# EUROPEAN PATENT APPLICATION

(11) **EP 4 411 372 A1**
(43) Date of publication of application: **07.08.2024**
(21) Application number: 22875396.8
(22) Date of filing: 18.03.2022
(51) Int. Cl.: G01N 31/00, G01N 33/18, G01N 30/88

(54) **GAS-LIQUID SEPARATOR, TOTAL ORGANIC CARBON METER, AND ANALYSIS SYSTEM**

(30) Priority: 30.09.2021 JP 2021160424
(71) Applicant: Shimadzu Corporation, Nakagyo-ku, Kyoto-shi, Kyoto 604-8511 (JP)
(72) Inventor: MAEDA, Kazuma, Kyoto-shi, Kyoto 604-8511 (JP)
(74) Representative: Müller-Boré & Partner Patentanwälte PartG mbB
(86) International application number: PCT/JP2022/012554
(87) International publication number: WO 2023/053513

(57) **Abstract**

A gas-liquid separator (50) includes a reception port (51a) to which a sample is supplied, a gas flow pipe (51, 52) through which gas in the sample supplied to the reception port (51a) is sent to the outside, a storage pipe (53) where liquid in the sample supplied to the reception port (51a) is stored, and a waste solution disposition port (54a) for disposition of liquid stored in the storage pipe (53) to the outside. The storage pipe (53) includes a first storage portion (53a) that communicates with the reception port (51a) and is arranged in an area vertically below the reception port (51a), a second storage portion (53b) that communicates with the waste solution disposition port (54a) and is arranged in an area vertically below the waste solution disposition port (54a), and a communication portion (53c) that allows communication between a vertically lower end of the first storage portion (53a) and a vertically lower end of the second storage portion (53b).

## Description

### TECHNICAL FIELD

The present disclosure relates to a gas-liquid separator, a total organic carbon analyzer, and an analysis system.

### BACKGROUND ART

A technique to measure an amount of total organic carbon (TOC) in a sample has conventionally been known as a technique to analyze a property of sample water (for example, Japanese Patent No. 6556699).

### CITATION LIST

### PATENT LITERATURE

PTL 1: Japanese Patent No. 6556699

### NON PATENT LITERATURE

NPL 1: Nobuyuki Kawasaki, Akio Imai, Kazuo Matsushige, Kazuhiro Komatsu, Fumikazu Ogishi, Masahito Yahata, Hirohisa Mikami, Takeshi Goto, "Investigation of molecular weight distribution of DOC in Lake Kasumigaura using size exclusion chromatography with TOC detector", The Japanese Society of Limnology, the 72nd annual conference proceedings, Session ID: 3C05, September 2007

### SUMMARY OF INVENTION

### TECHNICAL PROBLEM

Among apparatus TOC analyzers (total organic carbon analyzers) that measure an amount of TOC, there is what is called a wet oxidation TOC analyzer that measures an amount of TOC by irradiating a liquid sample injected into a reaction pipe with ultraviolet rays to oxidize an organic substance in the liquid sample to generate carbon dioxide gas (CO₂ gas) and sending generated CO₂ gas together with carrier gas as a gas sample into a detector.

The wet oxidation TOC analyzer has conventionally generally conducted batch measurement in which a sample is injected into the reaction pipe, thereafter injection of the sample into the reaction pipe is once suspended, the reaction pipe is irradiated with ultraviolet rays to oxidize an organic substance in the sample, and an amount of TOC is measured. In such conventional batch measurement, the amount of TOC cannot be measured continuously (in real time), and hence change over time in amount of TOC cannot continuously be measured while the sample is continuously kept sent to the reaction pipe.

In order to continuously measure change over time in amount of TOC in the wet oxidation TOC analyzer, a liquid sample irradiated with ultraviolet rays in the reaction pipe and a gas sample should continuously and appropriately be separated. Japanese Patent No. 6556699 described above, however, is silent about such a problem and measures therefor.

The present disclosure was made to solve the problem above, and an object of the present disclosure is to continuously and appropriately separate a sample containing gas and liquid into gas and liquid.

### SOLUTION TO PROBLEM

A gas-liquid separator according to the present disclosure is a gas-liquid separator that separates gas and liquid contained in a sample. The gas-liquid separator includes a reception port to which the sample is supplied, a gas flow pipe through which gas in the sample supplied to the reception port is sent to the outside, the gas flow pipe having one end communicating with the reception port, a storage pipe where liquid in the sample supplied to the reception port is stored, and a waste solution disposition port for disposition of liquid stored in the storage pipe to the outside. The storage pipe includes a first storage portion that communicates with the reception port, the first storage portion being arranged in an area vertically below the reception port, a second storage portion that communicates with the waste solution disposition port, the second storage portion being arranged in an area vertically below the waste solution disposition port, and a communication portion that allows communication between a vertically lower end of the first storage portion and a vertically lower end of the second storage portion.

A total organic carbon analyzer according to the present disclosure includes the gas-liquid separator described above, a mixer that mixes carrier gas into a liquid sample in prescribed cycles, an oxidation reactor arranged between the mixer and the gas flow pipe of the gas-liquid separator, the oxidation reactor irradiating a mixed sample that has passed through the mixer with ultraviolet rays, and a non-dispersive infrared absorption detector that detects a component in gas sent through the gas flow pipe of the gas-liquid separator.

An analysis system according to the present disclosure includes the total organic carbon analyzer described above and a liquid chromatograph that supplies a liquid sample to the mixer of the total organic carbon analyzer.

### ADVANTAGEOUS EFFECTS OF INVENTION

According to the present disclosure, a sample containing gas and liquid can continuously and appropriately be separated into gas and liquid.

### BRIEF DESCRIPTION OF DRAWINGS

Fig. 1 is a diagram schematically showing an exemplary configuration of an analysis system.
Fig. 2 is a diagram for illustrating exemplary principles of detection of a sample component by an LC unit.
Fig. 3 is a diagram showing correspondence between a solute size and an elution time period.
Fig. 4 is a diagram showing an exemplary configuration of a mixer, an oxidation reactor, and a gas-liquid separator.
Fig. 5 is a diagram schematically showing each step in analysis processing by the analysis system.
Fig. 6 is a diagram (No. 1) showing a configuration of a gas-liquid separator.
Fig. 7 is a diagram (No. 2) showing a configuration of a gas-liquid separator.
Fig. 8 is a diagram (No. 3) showing a configuration of a gas-liquid separator.
Fig. 9 is a diagram showing a configuration of a diagram (No. 4) showing a configuration of a gas-liquid separator.

### DESCRIPTION OF EMBODIMENTS

An embodiment of the present disclosure will be described in detail below with reference to the drawings. The same or corresponding elements in the drawings have the same reference characters allotted and description thereof will not be repeated.

### <System Configuration>

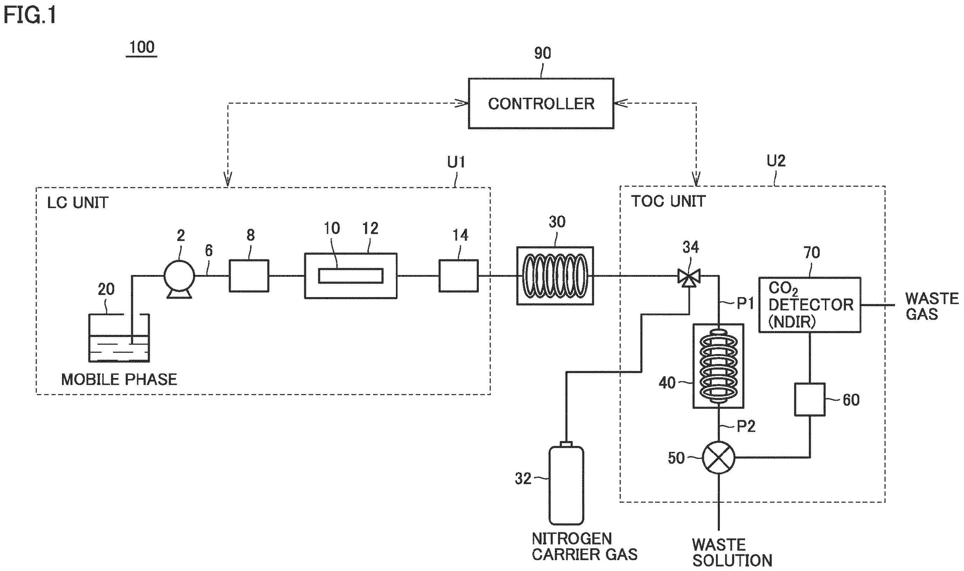

Fig. 1 is a diagram schematically showing an exemplary configuration of an analysis system 100 according to the present embodiment. Analysis system 100 includes a liquid chromatograph (LC) unit U1, a TOC unit U2, an inorganic carbonate removal and degassing system 30, a tank 32, and a controller 90.

LC unit U1 is an apparatus in which a sample to be analyzed is injected into a liquid mobile phase to pass through a column and a sample component is detected based on a difference in interaction between the sample (mobile phase) and a column filler (stationary phase).

LC unit U1 includes a container 20, a pump 2, an analysis flow channel 6, an autosampler 8, a column oven 12 in which a column 10 is accommodated, and a detector 14.

The mobile phase (ultrapure water, a phosphate buffer solution, or the like) is accommodated in container 20. Pump 2 suctions the mobile phase in container 20 and injects the mobile phase into analysis flow channel 6. On analysis flow channel 6, autosampler 8, column 10, and detector 14 are arranged from an upstream side toward a downstream side of a flow of the mobile phase.

Autosampler 8 injects a sample to be analyzed into the mobile phase in analysis flow channel 6. Column 10 separates the sample injected into the mobile phase. Detector 14 detects a sample component separated in column 10. A detector including a UV detector and a fluorescence detector connected in series can be employed as detector 14.

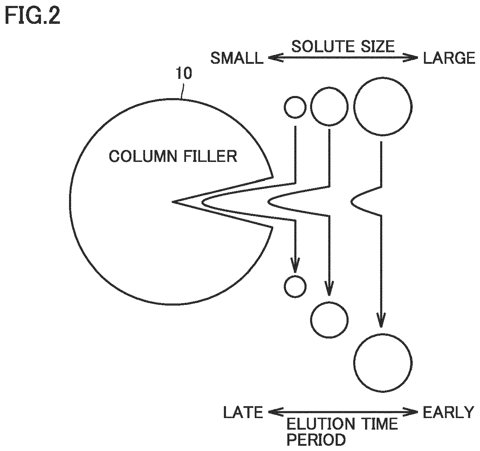

Fig. 2 is a diagram for illustrating exemplary principles of detection of a sample component by LC unit U1. Size exclusion chromatography (SEC) is adopted in LC unit U1 according to the present embodiment. Size exclusion chromatography refers to a method of detecting a solute component by utilizing such a property that a degree of permeation to the column filler varies depending on a difference in solute size (size of a molecule of a sample).

A column filler including small holes (pores) is carried on column 10 as the stationary phase. Depending on a difference in time period for which molecules are located in pores in the column filler, a time period until elution from column 10 (elution time period) is different. Specifically, small molecules migrate through column 10 while they are located deep inside the pores, and hence they require a long time period until they are eluted from column 10. As molecules are larger, however, they are less likely to enter the inside of the pores, and accordingly they are eluted from column 10 earlier.

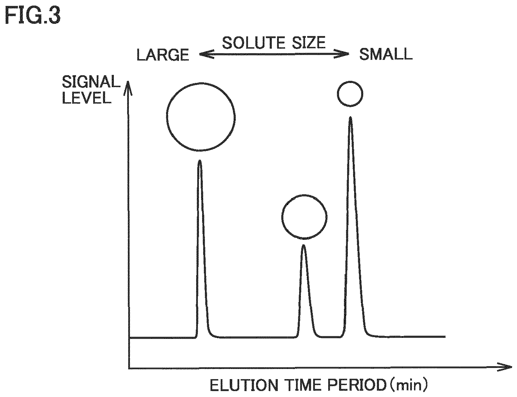

Fig. 3 is a diagram showing correspondence between a solute size and an elution time period. As shown in Fig. 3, relation is such that, as the solute size is larger, a migration path is shorter and the elution time period is shorter. Detector 14 in LC unit U1 detects the sample component based on the difference in elution time period as shown in Fig. 3.

Referring back to Fig. 1, inorganic carbonate removal and degassing system 30 is arranged between LC unit U1 and TOC unit U2. Inorganic carbonate removal and degassing system 30 removes an impurity (organic carbonate) which is not to be analyzed from a liquid sample that has passed through detector 14 in LC unit U1 and continuously sends the resultant liquid sample to TOC unit U2.

TOC unit U2 is configured to continuously measure an amount of TOC in the liquid sample continuously sent from LC unit U1 through inorganic carbonate removal and degassing system 30.

TOC unit U2 includes a mixer 34, an oxidation reactor 40, a gas-liquid separator 50, a dehumidifier 60, and a CO₂ detector 70. Mixer 34 and oxidation reactor 40 communicate with each other through a pipe P1 and oxidation reactor 40 and gas-liquid separator 50 communicate with each other through a pipe P2.

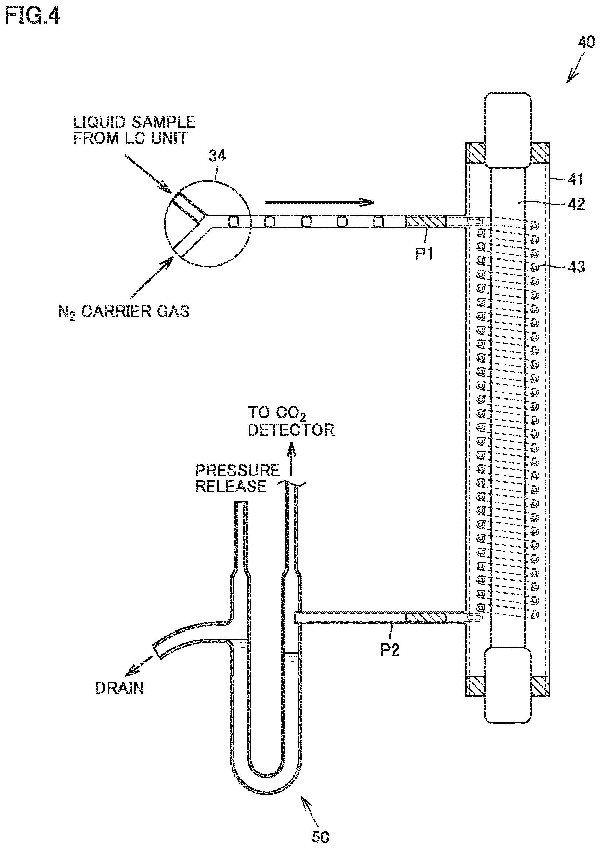

Fig. 4 is a diagram showing an exemplary configuration of mixer 34, oxidation reactor 40, and gas-liquid separator 50.

Mixer 34 mixes carrier gas (nitrogen gas) from tank 32 into the liquid sample continuously sent from LC unit U1 in prescribed cycles and supplies the mixed liquid sample to pipe P1. Oxidation reactor 40 is thus alternately supplied with the liquid sample in a form of a membrane and carrier gas.

Oxidation reactor 40 includes a UV lamp 42 accommodated in a UV oxidation pipe 41 and a helical reaction pipe 43 wound around UV lamp 42. The liquid sample in the form of the membrane and carrier gas supplied from mixer 34 are supplied from pipe P1 to reaction pipe 43, they pass through reaction pipe 43, and thereafter they are supplied to pipe P2.

The liquid sample in the form of the membrane and carrier gas are irradiated with ultraviolet rays generated by UV lamp 42 while they flow through reaction pipe 43. An organic substance contained in the liquid sample in the form of the membrane is thus oxidized to become CO₂ gas, which is mixed into carrier gas. Therefore, the liquid sample in the form of the membrane not containing the organic substance and a gaseous sample containing CO₂ gas and carrier gas are alternately sent from oxidation reactor 40 to pipe P2.

Gas-liquid separator 50 is configured to continuously and appropriately separate the liquid sample and the gaseous sample alternately sent from pipe P2. The liquid sample separated in gas-liquid separator 50 is disposed of, whereas the gaseous sample separated in gas-liquid separator 50 is sent to CO₂ detector 70. The configuration of gas-liquid separator 50 will be described in detail later.

Referring back to Fig. 1, the gaseous sample separated in gas-liquid separator 50 is sent to dehumidifier 60 and dehumidified therein, and thereafter the dehumidified gaseous sample is sent to CO₂ detector 70.

CO₂ detector 70 detects an amount of CO₂ gas contained in the sample treated in oxidation reactor 40, based on non-dispersive infrared absorption (NDIR) using infrared rays. A technique to detect CO₂ gas based on non-dispersive infrared absorption is a known technique. The gaseous sample detected by CO₂ detector 70 is emitted to the outside. A result of detection by CO₂ detector 70 is sent to controller 90. Controller 90 detects the amount of TOC in the sample based on the result of detection by CO₂ detector 70.

Controller 90 is typically a general personal computer which includes a display and is to be operated by an operator. Controller 90 controls operations of LC unit U1 and TOC unit U2. Specifically, controller 90 stores an analysis program for controlling operations of LC unit U1 and TOC unit U2 in analysis, and controls the operations, with analysis by LC unit U1 being in coordination with analysis by TOC unit U2 in accordance with the analysis program. Results of analysis by LC unit U1 and TOC unit U2 are shown, for example, on the display of controller 90.

When the operator has autosampler 8 filled with a sample to be analyzed and inputs a command to start analysis to controller 90, analysis processing by analysis system 100 is started.

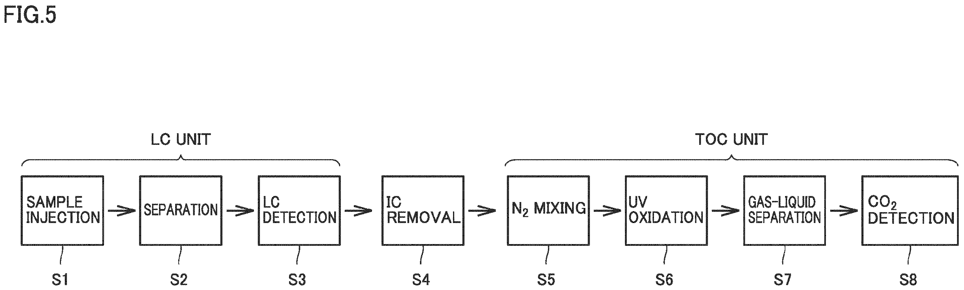

Fig. 5 is a diagram schematically showing each step in analysis processing by analysis system 100. In the analysis processing, the sample is processed in the order of steps S1 to S8.

Steps S1 to S3 are processing in LC unit U1. In step S1, the sample is injected from autosampler 8 into the mobile phase. In next step S2, processing for separating the sample injected into the mobile phase in column 10 is performed. In next step S3, the sample component separated in column 10 is detected by detector 14 (the UV detector and the fluorescence detector).

Next step S4 is processing in inorganic carbonate removal and degassing system 30. In step S4, processing for removal by inorganic carbonate removal and degassing system 30, of an impurity (inorganic carbonate: IC) in the liquid sample sent from detector 14 in LC unit U1 is performed.

Next steps S5 to S8 are processing in TOC unit U2. In step S5, mixer 34 mixes carrier gas into the liquid sample in prescribed cycles. The liquid sample in the form of the membrane and carrier gas are thus alternately supplied to oxidation reactor 40 as described above.

In next step S6, in oxidation reactor 40, the liquid sample in the form of the membrane and carrier gas supplied from mixer 34 are irradiated with ultraviolet rays. As described above, the organic substance in the liquid sample in the form of the membrane is thus oxidized to become CO₂ gas, which is then mixed into carrier gas (for example, N₂). Therefore, the liquid sample in the form of the membrane not containing the organic substance and the gaseous sample containing CO₂ gas and carrier gas are alternately sent to gas-liquid separator 50.

In next step S7, in gas-liquid separator 50, the liquid sample and the gaseous sample alternately sent from oxidation reactor 40 are separated from each other. The gaseous sample separated in gas-liquid separator 50 is sent to CO₂ detector 70.

In next step S8, CO₂ detector 70 detects an amount of CO₂ gas (an amount of TOC) in the gaseous sample separated in gas-liquid separator 50.

Thus, in analysis system 100 according to the present embodiment, combination of LC unit U1 and TOC unit U2 can achieve detection of a substance that cannot be detected by detector 14 (for example, the UV detector and the fluorescence detector) in LC unit U1, as the amount of CO₂ gas (the amount of TOC) in TOC unit U2.

### <Configuration of Gas-Liquid Separator 50>

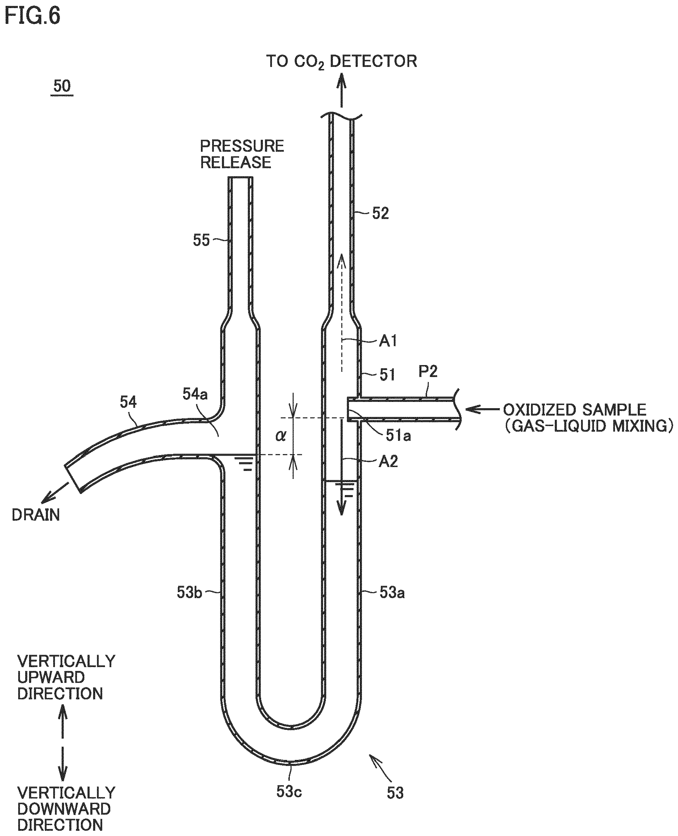

Fig. 6 is a diagram showing a configuration of gas-liquid separator 50. Gas-liquid separator 50 includes a reception port 51a, gas flow pipes 51 and 52, a storage pipe 53, a waste solution disposition port 54a, a drainage pipe 54, and a pressure release pipe 55.

The sample oxidized in oxidation reactor 40, that is, the liquid sample and the gaseous sample containing CO₂ gas as described above, is supplied through pipe P2 to reception port 51a.

Gas flow pipes 51 and 52 are pipes through which the gaseous sample supplied to reception port 51a is sent to external CO₂ detector 70. Gas flow pipe 51 has one end communicating with reception port 51a and the other end communicating with one end of gas flow pipe 52. Gas flow pipe 52 has the other end communicating with CO₂ detector 70. Gas flow pipe 51 is larger in inner diameter than gas flow pipe 52. In the present embodiment, as shown in Fig. 6, a ratio between the inner diameter of gas flow pipe 51 and the inner diameter of gas flow pipe 52 is approximately 2:1. The ratio between the inner diameter of gas flow pipe 51 and the inner diameter of gas flow pipe 52 is not limited to approximately 2:1. An optimal range of the ratio between the inner diameter of gas flow pipe 51 and the inner diameter of gas flow pipe 52 is, for example, a range from approximately 10:1 to approximately 1.5:1. In the present embodiment, the inner diameter of gas flow pipe 51 is substantially constant and the inner diameter of gas flow pipe 52 is also substantially constant.

Storage pipe 53 is a pipe for storage of a certain amount of liquid sample supplied to reception port 51a. Storage pipe 53 includes a first storage portion 53a, a second storage portion 53b, and a communication portion 53c.

First storage portion 53a and second storage portion 53b both extend in a vertical direction and are arranged as being horizontally adjacent to each other. First storage portion 53a has a vertically upper end communicating with reception port 51a, and is arranged in an area vertically below reception port 51a. Second storage portion 53b has a vertically upper end communicating with waste solution disposition port 54a, and is arranged in an area vertically below waste solution disposition port 54a. Communication portion 53c allows communication between a vertically lower end of first storage portion 53a and a vertically lower end of second storage portion 53b. Storage pipe 53 according to the present embodiment is formed in a U shape.

Waste solution disposition port 54a is a discharge port for disposition of liquid stored in storage pipe 53 to the outside through drainage pipe 54. Waste solution disposition port 54a is arranged at a position vertically downward at a prescribed distance α from reception port 51a. Pressure release pipe 55 is configured to allow communication between the vertically upper end of second storage portion 53b and the outside.

As set forth above, gas-liquid separator 50 according to the present embodiment includes gas flow pipes 51 and 52 through which the gaseous sample oxidized in oxidation reactor 40 is sent to CO₂ detector 70, storage pipe 53 having a volume that allows storage of a certain amount of liquid sample oxidized in oxidation reactor 40, and waste solution disposition port 54a and drainage pipe 54 for disposition of the liquid sample stored in storage pipe 53.

The sample oxidized in oxidation reactor 40 can thus continuously be separated into the gaseous sample and the liquid sample. In other words, by introduction of the sample continuously oxidized in oxidation reactor 40 together with carrier gas into gas-liquid separator 50, the liquid sample moves vertically downward from reception port 51a owing to self-weight as shown with an arrow A2 in Fig. 6 and is stored in storage pipe 53 in the U shape. Since the liquid sample stored in storage pipe 53 seals a flow channel through which the gaseous sample moves from reception port 51a to waste solution disposition port 54a and pressure release pipe 55, the gaseous sample can be supplied to CO₂ detector 70 without any leakage as shown with an arrow A1 in Fig. 6.

The volume of storage pipe 53 is set such that the whole liquid sample in storage pipe 53 does not flow to drainage pipe 54 due to a pressure generated in storage pipe 53 by a flow rate of carrier gas. The flow channel through which the gaseous sample moves from reception port 51a to waste solution disposition port 54a is thus appropriately sealed.

When a gas flow channel to CO₂ detector 70 is closed for some factor, the pressure in gas flow pipes 51 and 52 increases. Then, the whole liquid stored in storage pipe 53 is discharged from waste solution disposition port 54a to drainage pipe 54, so that the inside of gas flow pipes 51 and 52 opens to the atmosphere. A failure or liquid leakage due to abnormal increase in pressure in gas-liquid separator 50 can thus be prevented.

Waste solution disposition port 54a is provided at a position lower by prescribed distance α from reception port 51a. Since a position of a liquid level of the liquid sample stored in first storage portion 53a can thus be lower by prescribed distance α from reception port 51a, contact between the liquid sample stored in storage pipe 53 and the gaseous sample that newly enters gas-liquid separator 50 through reception port 51a can be avoided. Therefore, CO₂ gas to be analyzed can be prevented from being trapped again in the liquid sample. By minimizing prescribed distance α, an unnecessary dead volume of a gas layer from reception port 51a to the liquid level of the liquid sample stored in first storage portion 53a can be minimized.

Furthermore, gas-liquid separator 50 according to the present embodiment configured as above can continuously separate the sample into the gaseous sample and the liquid sample. Therefore, TOC unit U2 according to the present embodiment can conduct continuous measurement of the amount of TOC, rather than batch measurement as in the conventional example. Therefore, while information on the difference in elution time period obtained as a result of separation in column 10 in LC unit U1 is maintained also in TOC unit U2, the amount of TOC can be measured continuously (in a time series manner). Therefore, use as an SEC-TOC analyzer which is combination of LC unit U1 and TOC unit U2 can be realized.

Furthermore, gas-liquid separator 50 according to the present embodiment includes pressure release pipe 55 that allows communication between the vertically upper end of second storage portion 53b and the outside. Therefore, the vertically upper end of second storage portion 53b opens to the atmosphere, so that the liquid sample in second storage portion 53b can smoothly be discharged from waste solution disposition port 54a to drainage pipe 54.

### [First Modification]

CO₂ detector 70 of TOC unit U2 is configured to measure CO₂, with a signal value in a steady state when carrier gas not containing CO₂ is fed to CO₂ detector 70 at a constant flow rate and at a constant pressure being defined as a baseline. Therefore, during measurement with CO₂ detector 70, processing for detecting the baseline with UV irradiation by oxidation reactor 40 being temporarily suspended is periodically performed. When the flow rate or the pressure of carrier gas varies during detection of the baseline, the signal value of the baseline also varies, which may be a source of noise. Therefore, during measurement with CO₂ detector 70, the flow rate and the pressure of carrier gas are desirably always stable.

Formation of a liquid membrane in gas flow pipe 51 or 52 in gas-liquid separator 50 leads to a concern about temporary cut-off of the flow of carrier gas by the liquid membrane, variation in flow rate and pressure of carrier gas, and variation in baseline of CO₂ detector 70.

In particular, since the sample heated by irradiation with ultraviolet rays by UV lamp 42 in oxidation reactor 40 is sent to gas-liquid separator 50, vapor in the gaseous sample is cooled and some of the vapor becomes liquid while the gaseous sample flows through gas flow pipes 51 and 52 at a room temperature. Then, the liquid is attached to wall surfaces of gas flow pipes 51 and 52 as droplets, which tend to form the liquid membrane. When the pressure in gas flow pipes 51 and 52 increases to a certain level or higher after the liquid membrane is produced, the liquid membrane bursts by being pressed by the pressure or moves through gas flow pipes 51 and 52 as the liquid membrane. Repetition of such a phenomenon leads to a concern about instability of the baseline of CO₂ detector 70 and resultant noise.

In the present first modification, a spherical cooling volume is provided in a stage that immediately follows reception port 51a of gas-liquid separator 50. Thus, an area of the wall surface is increased to enhance a cooling effect and a structure where formation of the liquid membrane is less likely is obtained.

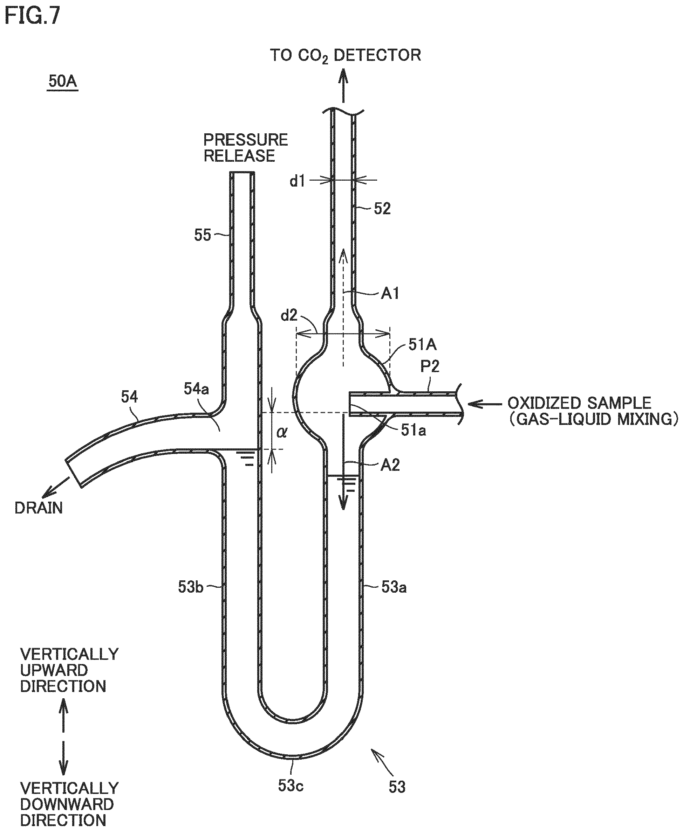

Fig. 7 is a diagram showing a configuration of a gas-liquid separator 50A according to the present first modification. Gas-liquid separator 50A according to the present first modification is obtained by change of gas flow pipe 51 in gas-liquid separator 50 according to the embodiment described above to a gas flow pipe 51A.

Gas flow pipe 51A has a portion immediately following reception port 51a formed in a spherical shape. An inner diameter d2 of gas flow pipe 51A around reception port 51a is thus set to a value considerably larger than an inner diameter d1 of gas flow pipe 52. In an example shown in Fig. 7, a ratio between inner diameter d1 of gas flow pipe 52 and inner diameter d2 of gas flow pipe 51A around reception port 51a is approximately 6:1. Formation of the liquid membrane around reception port 51a of gas flow pipe 51A is thus less likely. The ratio between inner diameter d1 of gas flow pipe 52 and inner diameter d2 of gas flow pipe 51A around reception port 51a is not limited to approximately 6:1. An optimal range of the ratio between inner diameter d1 of gas flow pipe 52 and inner diameter d2 of gas flow pipe 51A around reception port 51a is, for example, a range from approximately 20:1 to approximately 2.5:1.

Furthermore, the inner diameter of gas flow pipe 51 according to the embodiment described above is substantially constant, whereas the inner diameter of gas flow pipe 51A according to the present first modification is inclined to decrease with distance from reception port 51a. Gas flow pipe 51A according to the present first modification can thus be larger in area of the wall surface and can more efficiently cool the gaseous sample than gas flow pipe 51 according to the embodiment described above. Therefore, vapor in the gaseous sample can be liquefied in gas flow pipe 51A large in inner diameter so that the gaseous sample containing vapor is less likely to flow into gas flow pipe 52. Consequently, formation of the liquid membrane is less likely in gas flow pipe 52 small in inner diameter.

As set forth above, in gas-liquid separator 50A according to the present first modification, the spherical cooling volume is provided around reception port 51a of gas flow pipe 51A, so that formation of the liquid membrane in gas flow pipes 51A and 52 is less likely. The baseline of CO₂ detector 70 can thus be stabilized and measurement at high sensitivity can be conducted.

### [Second Modification]

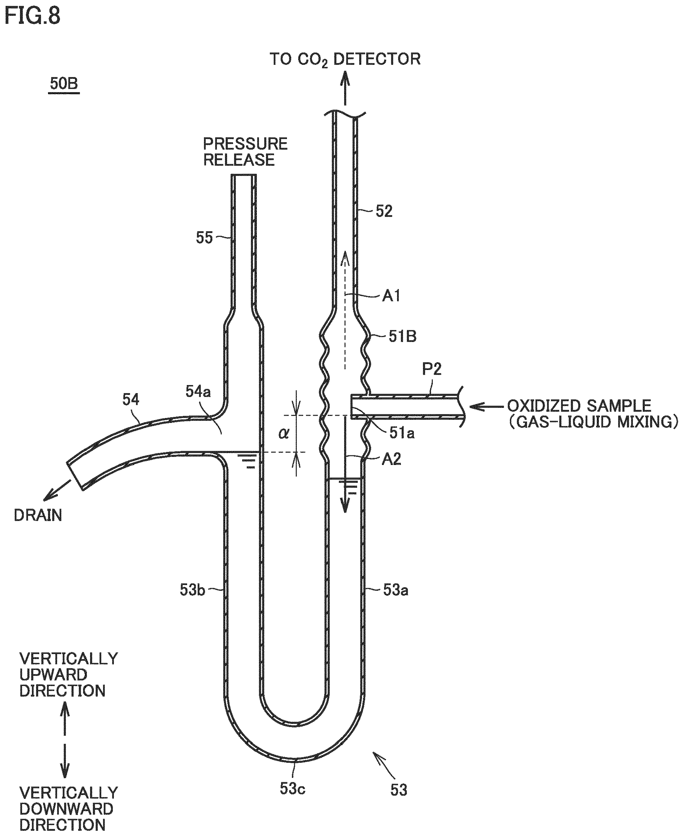

Fig. 8 is a diagram showing a configuration of a gas-liquid separator 50B according to the present second modification. Gas-liquid separator 50B according to the present second modification is obtained by change of gas flow pipe 51 of gas-liquid separator 50 according to the embodiment described above to a gas flow pipe 51B.

Gas flow pipe 51 according to the embodiment described above has a flat inner wall, whereas gas flow pipe 51B according to the present second modification has a corrugated inner wall. Gas flow pipe 51B according to the present second modification can thus be larger in area of the wall surface and can more efficiently cool the gaseous sample than gas flow pipe 51 according to the embodiment described above. Therefore, vapor in the gaseous sample can be liquefied in gas flow pipe 51B large in inner diameter so that formation of the liquid membrane is less likely in gas flow pipe 52 small in inner diameter.

An effect to cool gas flow pipe 51B may be enhanced by thus corrugating the inner wall of gas flow pipe 51B. By doing so, vapor can be liquefied at the inner wall of gas flow pipe 51B and the gaseous sample containing vapor is less likely to flow into gas flow pipe 52. Consequently, formation of the liquid membrane is less likely in gas flow pipe 52 small in inner diameter. The baseline of CO₂ detector 70 can thus be stabilized and measurement at high sensitivity can be conducted.

### [Third Modification]

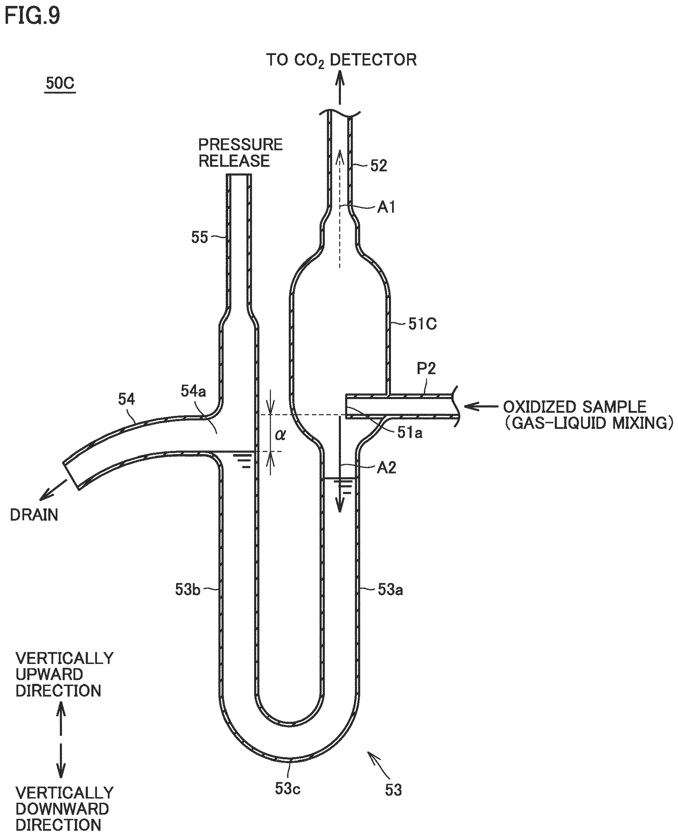

Fig. 9 is a diagram showing a configuration of a gas-liquid separator 50C according to the present third modification. Gas-liquid separator 50C according to the present third modification is obtained by change of gas flow pipe 51A according to the first modification described above to a gas flow pipe 51C.

Gas flow pipe 51A according to the first modification described above includes a spherical cooling volume, whereas gas flow pipe 51C according to the present third modification includes a spheroidal cooling volume. According to such a modification, formation of the liquid membrane in gas flow pipes 51C and 52 is less likely as in the first modification described above, and hence the baseline of CO₂ detector 70 can be stabilized and measurement at high sensitivity can be conducted.

### [Fourth Modification]

The embodiment described above illustrates an example in which a size exclusion (SEC) mode is adopted as a separation mode of LC unit U1 (see Fig. 2 described above). The separation mode of LC unit U1, however, is not limited to the size exclusion mode. For example, one of an adsorption mode, a distribution mode, and an ion exchange mode may be adopted as the separation mode of LC unit U1.

Though the embodiment described above illustrates an example in which LC unit U1 and TOC unit U2 are combined, LC unit U1 does not have to be provided. In other words, TOC unit U2 may measure all sample components, without separation of the sample component in LC unit U1.

### [Aspects]

The embodiment and the modifications thereof described above are understood by a person skilled in the art as specific examples of aspects below.

(Clause 1) A gas-liquid separator according to one aspect is a gas-liquid separator that separates gas and liquid contained in a sample. The gas-liquid separator includes a reception port to which the sample is supplied, a gas flow pipe through which gas in the sample supplied to the reception port is sent to outside, the gas flow pipe having one end communicating with the reception port, a storage pipe where liquid in the sample supplied to the reception port is stored, and a waste solution disposition port for disposition of liquid stored in the storage pipe to the outside. The storage pipe includes a first storage portion that communicates with the reception port, the first storage portion being arranged in an area vertically below the reception port, a second storage portion that communicates with the waste solution disposition port, the second storage portion being arranged in an area vertically below the waste solution disposition port, and a communication portion that allows communication between a vertically lower end of the first storage portion and a vertically lower end of the second storage portion.

According to the gas-liquid separator according to Clause 1, gas in the sample supplied to the reception port is sent to the gas flow pipe, whereas liquid in the sample moves vertically downward from the reception port owing to self-weight and is stored in the storage pipe. The liquid sample stored in the storage pipe seals a flow channel through which a gaseous sample moves from the reception port to the waste solution disposition port. The sample containing gas and liquid can thus continuously and appropriately be separated into gas and liquid.

(Clause 2) In the gas-liquid separator according to Clause 1, the waste solution disposition port is arranged at a position in a vertically downward direction at a prescribed distance from the reception port.

According to the gas-liquid separator according to Clause 2, a position of a liquid level of a liquid sample stored in the first storage portion can be lower by a prescribed distance from the reception port. Therefore, contact of gas that newly enters the gas-liquid separator through the reception port with liquid stored in the storage pipe can be avoided. Therefore, gas to be analyzed can be prevented from being trapped in liquid stored in the storage pipe.

(Clause 3) The gas-liquid separator according to Clause 1 or 2 further includes a pressure release pipe that allows communication between a vertically upper end of the second storage portion and the outside.

According to the gas-liquid separator according to Clause 3, the vertically upper end of the second storage portion communicates with the outside and opens to the atmosphere. Therefore, the liquid sample in the second storage portion can smoothly be discharged from the waste solution disposition port.

(Clause 4) In the gas-liquid separator according to any one of Clauses 1 to 3, the gas flow pipe includes a first gas flow pipe that communicates with the reception port and a second gas flow pipe that communicates with the first gas flow pipe. The first gas flow pipe is larger in inner diameter than the second gas flow pipe.

According to the gas-liquid separator according to Clause 4, the first gas flow pipe that communicates with the reception port is larger in inner diameter than the second gas flow pipe, so that liquid supplied to the reception port can be less likely to form a liquid membrane in the first gas flow pipe. The flow rate and the pressure of gas in the gas flow pipe can thus be stabilized.

(Clause 5) In the gas-liquid separator according to Clause 4, the inner diameter of the first gas flow pipe decreases with distance from the reception port. The inner diameter of the second gas flow pipe is substantially constant.

According to the gas-liquid separator according to Clause 5, the inner diameter of the first gas flow pipe is inclined to decrease with distance from the reception port, instead of being substantially constant, so that an area of a wall surface of the first gas flow pipe can be larger and gas that flows through the first gas flow pipe can more efficiently be cooled. Therefore, vapor in gas can be liquefied in the first gas flow pipe and vapor can be less likely to flow into the second gas flow pipe. Consequently, formation of the liquid membrane is less likely in the second gas flow pipe.

(Clause 6) In the gas-liquid separator according to any one of Clauses 1 to 3, the gas flow pipe includes a first gas flow pipe that communicates with the reception port and a second gas flow pipe that communicates with the first gas flow pipe. The second gas flow pipe has a flat inner wall. The first gas flow pipe has a corrugated inner wall.

According to the gas-liquid separator according to Clause 6, the inner wall of the first gas flow pipe is corrugated rather than being flat, so that the area of the wall surface of the first gas flow pipe can be larger and gas that flows through the first gas flow pipe can more efficiently be cooled. Therefore, vapor in gas can be liquefied in the first gas flow pipe and vapor can be less likely to flow into the second gas flow pipe. Consequently, formation of the liquid membrane is less likely in the second gas flow pipe.

(Clause 7) A total organic carbon analyzer according to one aspect includes the gas-liquid separator according to any one of Clauses 1 to 6, a mixer that mixes carrier gas into a liquid sample in prescribed cycles, an oxidation reactor arranged between the mixer and the gas flow pipe of the gas-liquid separator, the oxidation reactor irradiating a mixed sample that has passed through the mixer with ultraviolet rays, and a non-dispersive infrared absorption detector that detects a component in gas sent through the gas flow pipe of the gas-liquid separator.

According to this total organic carbon analyzer, the gas-liquid separator can continuously and appropriately separate a sample containing gas and liquid into gas and liquid. Therefore, the detector can continuously measure components in gas.

(Clause 8) An analysis system according to one aspect includes the total organic carbon analyzer according to Clause 7 and a liquid chromatograph that supplies a liquid sample to the mixer of the total organic carbon analyzer.

According to this analysis system, the total organic carbon analyzer can continuously detect components in a sample continuously supplied from the liquid chromatograph.

It should be understood that the embodiment disclosed herein is illustrative and non-restrictive in every respect. The scope of the present invention is defined by the terms of the claims rather than the description of the embodiment above and is intended to include any modifications within the scope and meaning equivalent to the terms of the claims.

### REFERENCE SIGNS LIST

2 pump; 6 analysis flow channel; 8 autosampler; 10 column; 12 column oven; 14 detector; 20 container; 30 inorganic carbonate removal and degassing system; 32 tank; 34 mixer; 40 oxidation reactor; 41 UV oxidation pipe; 42 lamp; 43 reaction pipe; 50, 50A, 50B, 50C gas-liquid separator; 51, 51A, 51B, 51C, 52 gas flow pipe; 51a reception port; 53 storage pipe; 53a first storage portion; 53b second storage portion; 53c communication portion; 54 drainage pipe; 54a waste solution disposition port; 55 pressure release pipe; 70 CO₂ detector; 60 dehumidifier; 90 controller; 100 analysis system; P1, P2 pipe; U1 LC unit; U2 TOC unit

## Claims

1. A gas-liquid separator that separates gas and liquid contained in a sample, the gas-liquid separator comprising:
a reception port to which the sample is supplied;
a gas flow pipe through which gas in the sample supplied to the reception port is sent to outside, the gas flow pipe having one end communicating with the reception port;
a storage pipe where liquid in the sample supplied to the reception port is stored; and
a waste solution disposition port for disposition of liquid stored in the storage pipe to the outside, wherein
the storage pipe includes
a first storage portion that communicates with the reception port, the first storage portion being arranged in an area vertically below the reception port,
a second storage portion that communicates with the waste solution disposition port, the second storage portion being arranged in an area vertically below the waste solution disposition port, and
a communication portion that allows communication between a vertically lower end of the first storage portion and a vertically lower end of the second storage portion.

2. The gas-liquid separator according to claim 1, wherein
the waste solution disposition port is arranged at a position in a vertically downward direction at a prescribed distance from the reception port.

3. The gas-liquid separator according to claim 1, further comprising a pressure release pipe that allows communication between a vertically upper end of the second storage portion and the outside.

4. The gas-liquid separator according to claim 1, wherein
the gas flow pipe includes
a first gas flow pipe that communicates with the reception port, and
a second gas flow pipe that communicates with the first gas flow pipe, and
the first gas flow pipe is larger in inner diameter than the second gas flow pipe.

5. The gas-liquid separator according to claim 4, wherein
the inner diameter of the first gas flow pipe decreases with distance from the reception port, and
the inner diameter of the second gas flow pipe is substantially constant.

6. The gas-liquid separator according to claim 1, wherein
the gas flow pipe includes
a first gas flow pipe that communicates with the reception port, and
a second gas flow pipe that communicates with the first gas flow pipe, the second gas flow pipe has a flat inner wall, and
the first gas flow pipe has a corrugated inner wall.

7. A total organic carbon analyzer comprising:
the gas-liquid separator according to claim 1;
a mixer that mixes carrier gas into a liquid sample in prescribed cycles;
an oxidation reactor arranged between the mixer and the gas flow pipe of the gas-liquid separator, the oxidation reactor irradiating a mixed sample that has passed through the mixer with ultraviolet rays; and
a non-dispersive infrared absorption detector that detects a component in gas sent through the gas flow pipe of the gas-liquid separator.

8. An analysis system comprising:
the total organic carbon analyzer according to claim 7; and
a liquid chromatograph that supplies a liquid sample to the mixer of the total organic carbon analyzer.
